Europäisches Patentamt

European Patent Office

Office européen des brevets

(11) Veröffentlichungsnummer: **0 243 521**

**A1**

# (12) EUROPÄISCHE PATENTANMELDUNG

(21) Anmeldenummer: 86105940.0

(22) Anmeldetag: 30.04.86

(51) Int. Cl.⁴: **A61K 9/20** , A61K 9/50

(43) Veröffentlichungstag der Anmeldung:
04.11.87 Patentblatt 87/45

(84) Benannte Vertragsstaaten:
AT BE CH DE FR GB IT LI LU NL SE

(71) Anmelder: **Thorn, Werner Prof. Dr.**
**Am Langenzug 9**
**D-2000 Hamburg 76(DE)**

(72) Erfinder: **Thorn, Werner Prof. Dr.**
**Am Langenzug 9**
**D-2000 Hamburg 76(DE)**

(74) Vertreter: **Dipl.-Ing. H. Hauck Dipl.-Phys. W.**
**Schmitz Dipl.-Ing. E. Graalfs Dipl.-Ing. W.**
**Wehnert Dr.-Ing. W. Döring**
**Neuer Wall 41**
**D-2000 Hamburg 36(DE)**

(54) **Oral verabreichbares Präparat.**

(57) Oral verabreichbares Präparat für Menschen oder Tiere, enthaltend eine Wirksubstanz, die eine sich in der Bildung von Plasmapolypen manifestierende schleimhautschädigende Nebenwirkung hat, und eine zur Bekämpfung der Nebenwirkung geeignete Hilfssubstanz aus der Gruppe Intermediärprodukte des Glucoseabbaues und Glieder des Citratcyklus, vorzugsweise Natriumpyruvat, dadurch gekennzeichnet, daß das Präparat zum Verabreichen in gelöster Form bestimmt ist und in Form von trocken lagerfähigen, gut wasserlöslichen Tabletten vorliegt, in welchen die Wirksubstanz und die Hilfssubstanz im Gemisch als wasserlösliche Pulver enthalten sind und die Pulverteilchen von einer festen oder halbfesten, an der Atmosphäre lagerfähigen, wasserlöslichen, reaktionsträgen und physiologisch unbedenklichen Schutzsubstanz weitgehend eingehüllt und an Reaktionen mit der Umgebung oder miteinander gehindert sind.

EP 0 243 521 A1

## Oral verabreichbares Präparat

Die Erfindung betrifft ein Präparat nach dem Oberbegriff des Anspruches 1.

Präparate der angegebenen Art sind aus der DE-PS 28 58 001 bekannt.

Viele Wirksubstanzen und Hilfssubstanzen reagieren mehr oder weniger schnell mit Feuchtigkeit oder Luft oder untereinander, so daß die Lagerfähigkeit begrenzt ist. Um Interaktionen zwischen verschiedenen Substanzen auszuschalten, liegt es nahe, die Wirksubstanz und die Hilfssubstanz getrennt zu halten, beispielsweise in einer sog. Kombinationspackung. Bei der Benutzung derartiger Kombinationspräparate können leicht Irrtümer unterlaufen; so kann insbesondere die Hilfssubstanz mit einem so großen zeitlichen Abstand nach der Wirksubstanz eingenommen werden, daß Schleimhautschäden im Magen bereits eingetreten sind. Auch ist das Hantieren mit verschiedenen Substanzen umständlich.

Die Erfindung geht von der Aufgabe aus, ein Präparat der angegebenen Art zu schaffen, das bequem und ohne die Gefahr von Irrtümern eingenommen werden kann, gut lagerfähig ist und eine optimale Schutzfunktion der Hilfssubstanz sicherstellt.

Nach der Erfindung wird diese Aufgabe mit dem Präparat nach dem Anspruch 1 gelöst.

Bei dem erfindungsgemäßen Präparat liegen die Wirksubstanz und die Hilfssubstanz als Pulver im Gemisch miteinander vor, so daß die beschriebenen Verabreichungsfehler nicht vorkommen können. Das Präparat besteht aus leicht wasserlöslichen Tabletten und ist zur Verabreichung in gelöster Form bestimmt und geeignet. Dadurch sind gleichmäßige Konzentrationen und Verteilungen der Substanzen und eine optimale Wirksamkeit der Hilfssubstanz sichergestellt. Die leichte Löslichkeit bietet ferner den Vorteil, daß auch bei (z.B. versehentlichem) Verabreichen in ungelöster Form die Substanzen im Magen rasch gelöst werden und die Hilfssubstanz überall gleichzeitig mit der Wirksubstanz freigesetzt wird und ihre Schutzwirkung rasch entfalten kann. Der Begriff "Pulver" soll auch Granulate einschließen.

Die beschriebenen Vorteile werden bei dem erfindungsgemäßen Präparat erzielt, ohne daß dafür eine geringere Haltbarkeit oder eine verminderte Lagerfähigkeit in Kauf genommen werden müßte. Da die Pulverteilchen der reaktionsfähigen Substanzen von der reaktionsträgen Schutzsubstanz weitgehend umhüllt und gegeneinander isoliert sind, sind Reaktionen der Substanzen sowohl mit der Umgebung als auch untereinander weitgehend ausgeschlossen, so daß das erfindungsgemäße Präparat bei üblichen Lagerbedingungen, beispielsweise in verschlossenen Metall-oder Glasrohren, sehr lange Zeit lagerfähig ist und seine Wirksamkeit voll beibehält.

Vorzugsweise enthalten die Tabletten zusätzlich wenigstens ein Brausemittel, um das Auflösen zu beschleunigen. Es können dadurch Auflösezeiten von zum Beispiel nur 30 Sekunden erzielt werden. Als Brausemittel ist insbesondere, wie an sich bekannt, ein Gemisch aus Natriumbicarbonat und einer physiologisch unbedenklichen, wasserlöslichen, festen organischen Säure geeignet. Derartige Brausemittel können bequem in Form Pulvern in den Tabletten mit verpreßt werden.

Als Schutzsubstanz ist insbesondere Polyethylenglycol im Molekulargewichtsbereich von etwa 2000 bis 5000 geeignet. Bei diesen Molekulargewichten ist Polyethylenglycol noch leicht und gut wasserlöslich, jedoch nicht mehr in störendem Ausmaß vom menschlichen Verdauungstrakt resorbierbar; es bleibt physiologisch unwirksam und wird vom Organismus praktisch vollständig wieder ausgeschieden. Besonders gut geeignet ist Polyethylenglycol einer Molekulargewichtsfraktion um das Molekulargewicht 4000; diese Substanz ist im Handel erhältlich.

Als Wirksubstanz kann in dem Präparat insbesondere ein Antirheumamittel vorliegen. Viele Antirheumamittel sind stark schleimhautschädigend; demgemäß ist die vorliegende Erfindung gerade für derartige Mittel besonders vorteilhaft. Ganz besonders gilt dies für die hochwirksamen, aber auch besonders stark schleimhautschädigenden Antirheumamittel aus der Gruppe Salicylsäure, Acetylsalicylsäure, Diclofenac und Derivate davon.

Zur Herstellung des erfindungsgemäßen Präparats wird mit Vorteil ein Verfahren verwendet, das dadurch gekennzeichnet ist, daß vor dem Pressen der Tabletten die Schutzsubstanz in zerkleinerter Form eingemischt wird. Insbesondere bei halbfester Konsistenz der Schutzsubstanz kann dabei ein verhältnismäßig grober Zerkleinerungsgrad ausreichen; beim Tablettieren oder Granulieren fließt dann die Schutzsubstanz zwischen und um die Teilchen der übrigen Substanzen. Eine besonders wirksame Schutzfunktion der Schutzsubstanz läßt sich sicherstellen, wenn vor dem Pressen der Tabletten die zu vermischenden und zu verpressenden Substanzen gesondert mit der Schutzsubstanz granuliert werden.

Vorzugsweise wird die Schutzsubstanz als Pulver zugegeben; dann ist schon beim Granulieren oder Tablettieren bei geringen Preßdrücken eine gute Verteilung der Schutzsubstanz sichergestellt.

Es ist sehr zweckmäßig, die Tabletten aus höchstmöglich wasserfreien Bestandteilen zu pressen. Es hat sich gezeigt, daß dann das tablettenförmige Präparat besonders haltbar und lange lagerfähig ist. Um schädliche Einwirkungen von Feuchtigkeit auszuschließen, ist es vorteilhaft, die Herstellung des Präparats unter höchstmöglich trockenen Bedingungen durchzuführen.

**Ansprüche**

1. Oral verabreichbares Präparat für Menschen oder Tiere, enthaltend eine Wirksubstanz, die eine sich in der Bildung von Plasmapolypen manifestierende schleimhautschädigende Nebenwirkung hat, und eine zur Bekämpfung der Nebenwirkung geeignete Hilfssubstanz aus der Gruppe Intermediärprodukte des Glucoseabbaues und Glieder des Citratcyklus, vorzugsweise Natriumpyruvat, dadurch gekennzeichnet, daß das Präparat zum Verabreichen in gelöster Form bestimmt ist und in Form von trocken lagerfähigen, gut wasserlöslichen Tabletten vorliegt, in welchen die Wirksubstanz und die Hilfssubstanz im Gemisch als wasserlösliche Pulver enthalten sind und die Pulverteilchen von einer festen oder halbfesten, an der Atmosphäre lagerfähigen, wasserlöslichen, reaktionsträgen und physiologisch unbedenklichen Schutzsubstanz weitgehend eingehüllt und an Reaktionen mit der Umgebung oder miteinander gehindert sind.

2. Präparat nach Anspruch 1, dadurch gekennzeichnet, daß die Tabletten zusätzlich wenigstens ein Brausemittel enthalten.

3. Präparat nach Anspruch 2, dadurch gekennzeichnet, daß als Brausemittel ein Gemisch aus Natriumbicarbonat und einer physiologisch unbedenklichen, wasserlöslichen, festen organischen Säure vorliegt.

4. Präparat nach einem der vorhergehenden Ansprüche, dadurch gekennzeichnet, daß als Schutzsubstanz Polyethylenglycol im Molekulargewichtsbereich von etwa 2500 bis 5000 vorliegt.

5. Präparat nach Anspruch 4, dadurch gekennzeichnet, daß das Polyethylenglycol einer Molekulargewichtsfraktion um das Molekulargewicht 4000 angehört.

6. Präparat nach einem der vorhergehenden Ansprüche, dadurch gekennzeichnet, daß als Wirksubstanz ein Antirheumamittel vorliegt.

7. Präparat nach Anspruch 6, dadurch gekennzeichnet, daß eine Wirksubstanz aus der Gruppe Salicylsäure, Acetylsalicylsäure, Diclofenac und Derivate davon vorliegt.

8. Verfahren zum Herstellen des Präparats nach einem der vorhergehenden Ansprüche, dadurch gekennzeichnet, daß vor dem Pressen der Tabletten die Schutzsubstanz in zerkleinerter Form zugegeben wird.

9. Verfahren nach Anspruch 8, dadurch gekennzeichnet, das vor dem Pressen der Tabeletten die zu vermischenden und zu verpressenden Substanzen gesondert mit Schutzsubstanz granuliert werden.

10. Verfahren nach Anspruch 8 oder 9, dadurch gekennzeichnet, daß die Schutzsubstanz als Pulver zugegeben wird.

11. Verfahren nach einem der Ansprüche 8 bis 10, dadurch gekennzeichnet, daß die Tabletten aus höchstmöglich wasserfreien Bestandteilen gepreßt werden.

12. Verfahren nach einem der Ansprüche 8 bis 11, dadurch gekennzeichnet, daß es unter höchstmöglich trockenen Bedingungen durchgeführt wird.

# EUROPÄISCHER RECHERCHENBERICHT

**Europäisches Patentamt**

EP 86 10 5940

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,Y | DE-C-2 858 001 (THORN) <br> * Seite 1, Zeilen 1-58 * | 1-12 | A 61 K 9/20 <br> A 61 K 9/50 |
| Y | FR-A-2 350 102 (BEECHAM) <br> * Seite 1, Zeilen 1-34; Seite 3, Zeile 2 - Seite 4, Zeile 21; Seite 7, Beispiel 5; Ansprüche 10-17 * | 1-12 | |
| Y | US-A-2 540 253 (GAKENHEIMER) <br> * Spalte 1, Zeilen 28-38; Spalte 4, Beispiel 4 * | 1,4-12 | |
| A | FR-A-2 293 438 (TAKEDA CHEM. IND.) <br> * Seite 1, Zeilen 18-32; Seite 2, Zeilen 6-32; Seite 4, Zeilen 37-40; Seite 5, Formulierung 2 * | | RECHERCHIERTE SACHGEBIETE (Int. Cl.4) <br><br> A 61 K |
| A | DE-A-2 251 199 (FARBWERKE HOECHST) <br> * Seite 2, Zeile 24 - Seite 4, Zeile 2; Seite 5, Zeile 1 - Seite 8, Ende * | | |

-----

Der vorliegende Recherchenbericht wurde für alle Patentansprüche erstellt.

| Recherchenort | Abschlußdatum der Recherche | Prüfer |
|---|---|---|
| DEN HAAG | 08-12-1986 | BENZ K.F. |